# EUROPEAN PATENT SPECIFICATION

(11) **EP 0 668 923 B1**
(45) Date of publication and mention of the grant of the patent: **12.05.1999**
(21) Application number: 93922456.4
(22) Date of filing: 08.10.1993
(51) Int. Cl.: C12P 21/08, C12N 5/18, C12N 5/20, A61K 39/395

(54) **MONOCLONAL ANTIBODY**
MONOKLONALER ANTIKÖRPER
ANTICORPS MONOCLONAL

(30) Priority: 09.10.1992 AU PL9252/17
(43) Date of publication of application: 30.08.1995
(73) Proprietor: AMRAD CORPORATION LIMITED, Kew, VIC 3101 (AU)
(72) Inventor: METCALF, Donald, Balwyn, VIC 3103 (AU); NICOLA, Nicos Antony, Mont Albert, VIC 3128 (AU); BOYD, Andrew Wallace, Moonee Ponds, VIC 3039 (AU); LAYTON, Judith Eleanor, Clifton Hill, VIC 3068 (AU); WYCHERLEY, Kaye, Box Hill, VIC 3128 (AU)
(74) Representative: Dzieglewska, Hanna Eva
(86) International application number: AU9300516
(87) International publication number: WO9409149

(56) References cited:
- WO-A-94/11404
- AU-A- 6 189 690
- DATABASE MEDLINE FILE SERVER STN KARLSRUHE ABSTRACT 94003211, NICOLA ET AL: "NEUTRALIZING AND NONNEUTRALIZING MONOCLONAL ANTIBODIES TO THE HUMAN GRANULOCYTE-MACROPHAGE COLONY-STIMULATING FACTOR RECEPTOR ALPHA-CHAIN" XP002000610
- The EMBO Journal, Vol. 8, No. 12, 1989, pp. 3667-3676, GEARING D.P. et al., "Expression Cloning of a Receptor for Human Granulocyte-Macrophage Colony-Stimulating Factor", whole document.
- Proc. Natl. Acad. Sci. USA, Vol. 88, 1991, pp. 5082-5086, KITAMURA et al., "Reconstitution of Functional Receptors for Human Granulocyte/Macrophage Colony-Stimulating Factor (GM-CSF): Evidence that the Protein Encoded by the AIC2B cDNA is a Subunit of the Murine GM-CSF Receptor", whole document.
- Proc. Natl. Acad. Sci. USA, Vol. 87, 1990, pp. 4670-4674, METCALF D. et al., "Low-Affinity Placenta-Derived Receptors for Human Granulocyte-Macrophage Colony-Stimulating Factor Can Deliver a Proliferative Signal to Murine Hemopoietic Cells".
- Nature, Vol. 256, 1975, pp. 495-497, KOEHLER, G. and MILSTEIN C., "Continuous Cultures of Fused Cells Secreting Antibody of Predefined Specificity".
- Proc. Natl. Acad. Sci. USA, Vol. 87, 1990, pp. 9655-9659, HAYASHIDA K. et al., "Molecular Cloning of a Second Subunit of the Receptor for Human Granulocyte-Macrophage Colony-Stimulating Factor (GM-CSF): Reconstitution of a High-Affinity GM-CSF Receptor".
- The Journal of Immunology, Vol. 144, 1990, pp. 2184-2189, BROWN C. et al., "Two Neutralizing Monoclonal Antibodies Against Human Granulocyte-Macrophage Colony-Stimulating Factor Recognize the Receptor Binding Domain of the Molecule".
- BLOOD, vol. 73, no. 7, 15.05.1989, p. 1786-1793, Sherr et al.
- PROC. NATL. ACAD. SCI. USA, vol. 81, July 1984, p. 3998-4002, Geysen et al.

## Description

This invention relates to monoclonal antibodies specific for the receptor for human granulocyte-macrophage colony stimulating factor (hGM-CSF), and to compositions and methods utilizing these antibodies.

### Background and Prior Art

Granulocyte-macrophage colony-stimulating factor (GM-CSF) is a growth and differentiation factor for a variety of haemopoietic progenitor cells (including those for neutrophils, macrophages, eosinophils, megakaryocytes and erythroid cells) and can also functionally activate mature neutrophils, eosinophils and macrophages (1-4). All of the actions of GM-CSF are thought to be mediated through the interaction of GM-CSF with specific cell surface receptors. These receptors consist of a specific α-chain (PCT/AU90/00342, published as WO 91/02063) (5), which binds GM-CSF with low-affinity, and a common β-chain, which by itself does not bind GM-CSF with detectable affinity, and is shared by the α-chains for the interleukin-3 and interleukin-5 receptors (6,7). The α-β complex generates high-affinity binding sites for GM-CSF, and is required for cell signalling (8). However, the mechanism of conversion of the low affinity receptor to high affinity is not yet understood.

Since murine models of excess GM-CSF production (9,10) have indicated that a variety of disease states can result from over-production of GM-CSF and consequent macrophage accumulation, and since clinical trials of GM-CSF have indicated that some disease states can be exacerbated by the action of GM-CSF (11), we believe that antibodies that inhibit GM-CSF action may have clinical utility. Moreover, since several human myeloid leukaemias have been shown both to possess GM-CSF receptors and to respond to GM-CSF by proliferation (12-16), we believe that such antibodies could be used to suppress leukaemic cell proliferation or to target leukaemic cells for antibody-mediated killing. Finally we believe that identification of the antibody epitope on the GM-CSF receptor α-chain recognized by neutralizing antibody will aid in the design of selective peptide and non-peptide antagonists of GM-CSF action.

We have generated a panel of monoclonal antibodies that specifically recognize the human GM-CSF receptor α-chain, and one of these inhibits the capacity of GM-CSF to bind to its cellular receptor and to biologically stimulate cells that bear GM-CSF receptors. Surprisingly, this inhibition occurs on cells that bear either the α-chain of the receptor alone (FD-A5 cells), or which bear the high-affinity α-β complex (AML 193 cells, human bone marrow cells).

### Summary of the Invention

According to a first aspect, the invention provides monoclonal antibodies specific for hGM-CSF receptor, which antibodies have the ability to inhibit the biological activity of hGM-CSF. Preferably the monoclonal antibodies of the invention are able to bind to both the high affinity and to the low-affinity GM-CSF receptor.

In a preferred embodiment, the monoclonal antibody is KI-2B7-17-A.

According to a second aspect, the invention provides hybridoma cell lines producing these monoclonal antibodies.

According to a third aspect, the invention provides therapeutic compositions comprising a monoclonal antibody according to the invention, together with a pharmaceutically acceptable carrier.

According to a fourth aspect of the invention, there is provided a method of treatment of a GM-CSF dependent condition, comprising the step of administering to a patient in need of such treatment an effective amount of a monoclonal antibody according to the invention, optionally together with a pharmaceutically-acceptable carrier. It is contemplated that methods of treatment either by suppression of leukaemic cell proliferation, or by targeting of leukaemic cells for antibody-mediated killing are within the scope of the invention. This method of the invention is applicable to both acute and chronic myeloid leukaemia, and to other leukaemias which are influenced by GM-CSF.

In a particularly preferred embodiment, the method of the invention provides a method of mitigation of the side-effects of treatment with GM-CSF, comprising the step of administering to a patient in need of such treatment an effective amount of a monoclonal antibody of the invention, optionally together with a pharmaceutically-acceptable carrier.

In a further aspect, the invention provides a method of screening of selective antagonists to GM-CSF, comprising the step of utilizing an antibody of the invention to identify the specific epitope on GM-CSF receptor recognised by said antibody, and testing putative antagonists of GM-CSF action for ability to bind to said epitope. The antagonists may be either peptides, or non-peptide molecules.

Using the methods described herein, screening of a relatively small number of hybridoma clones revealed 15 candidate clones, of which one surprisingly produced a monoclonal antibody having inhibitory activity against high-affinity hGM-CSF receptor. Thus hybridoma clones having the required specific activity can be produced at high frequency, and it is considered that the skilled person would readily be able to utilise the methods described herein in order to produce neutralising monoclonal antibodies.

Similarly, methods for producing "humanised" forms of monoclonal antibodies, or for producing antigen-binding fragments of monoclonal antibodies, by the use of genetic engineering techniques are well-known in the art. Thus such humanised forms or antigen-binding fragments are also to be clearly understood to be within the scope of the invention.

### Detailed Description of the Invention

The invention will now be described by way of reference only to the following non-limiting examples, and to the accompanying drawings, in which
Figure 1 shows the results of immunodepletion assays in which the ability of monoclonal antibodies to deplete solubilized hGM-CSF receptor α-chain from solution was tested;
Figure 2 summarises results of immunofluroescence detection of hGM-CSF receptor α-chain on FDA5 cells;
Figure 3 shows results of direct binding inhibition studies, showing dose-dependent inhibition by a representative antibody of the invention of binding of GM-CSF to receptor;
Figure 4 shows suppression of specific binding of GM-CSF to receptors by the antibody of the invention;
Figure 5 shows that only one of the five monoclonal antibodies tested had the ability to directly inhibit binding of hGM-CSF to solubilised receptor;
Figure 6a shows results of binding studies to determine the binding affinity of an antibody of the invention to hGM-CSF receptor;
Figure 6b shows corresponding Scatchard plots;
Figure 7 shows the kinetic association and dissociation rates of binding of an antibody of the invention to hGM-CSF receptor.
Figure 8 shows inhibition by an antibody of the invention of colony formation by human bone marrow cells in response to hGM-CSF;
Figure 9 shows that this inhibition is dose-dependent;
Figure 10 illustrates the ability of an antibody of the invention to inhibit growth *in vitro* of an hGM-CSF dependent cell line; and
Figure 11 shows inhibition of stimulation by hGM-CSF of a cell line transfected with hGM-CSF receptor α-chain.

In the following examples, the invention is described in detail with reference to monoclonal antibody produced by the hybridoma cell line KI-2B7-17-A. A sample of this hybridoma cell line was deposited under the Budapest Treaty in the Public Health Laboratory Service Centre for Applied Microbiology and Research European Collection of Animal Cell Cultures, Porton Down, Salisbury, Wiltshire, SP4 OJG, U.K. on 23 September 1992, under Accession Number 92092317.

Abbreviations used herein are as follows:
- DME: Dulbecco's modified Eagle's medium
- FCS: foetal calf serum
- FITC: Fluorescein isothiocyanate
- GM-CSF: granulocyte-macrophage colony stimulating factor
- ICAM-1: Intercellular adhesion molecule-1
- SAMIg: Sheep anti-mouse immunoglobulin
All of the cell lines referred to herein are publicly available, or have been previously described in the literature.

### Example 1 Hybridoma Production

In initial studies, mouse FDC-P1 cells transfected with the human GM-CSF receptor α-chain (FDA5 cells) (17) were injected into syngeneic BALB/c recipients, in the expectation that this would result in more specific immune responses. No successful fusions resulted with this protocol. Since our prior observations had shown that allogeneic responses enhanced the immune response to other cell surface antigens, we immunized CBA/J mice with allogenic FDA5 cells. The mice received weekly intraperitoneal injections of 2-3x10⁷ viable FDA5 cells for four weeks, and sera were assayed in the immunodepletion assay (see below). The mice were then rested for at least four weeks before a final immunization. Four days after this final boost the animals were killed by cervical dislocation and the spleens removed. Single cell suspensions were prepared and washed in serum-free Dulbecco's modified Eagle's medium (DME). The spleen cells were fused with NS-1 myeloma cells in the presence of 50% (w/v) polyethylene glycol 4000 (Boehringer-Mannheim) (18).

The cells were gently resuspended in DME/15% (v/v) foetal calf serum (FCS) 10% (v/v) conditioned medium from the P33D18 macrophage cell line (as a source of interleukin-6), and cultured in 96 well Falcon microwell flat bottom trays at 2x10⁵ cells per well. The cells were fed with a standard selective medium containing hypoxanthine, aminopterin and thymidine (HAT medium) after overnight culture, and thereafter every three days. After 7-14 days, developing hybridoma clones were observed in the wells and supernatants were harvested for testing.

### Example 2 Screening of Hybridoma Clones

### a) Analysis of COS 7 Cell Transfectants by Immunofluorescence.

COS 7 cells (ATCC CRL 9589) were electroporated with expression plasmids encoding the α-chain of the human GM-CSE receptor (5) or the cell surface molecule ICAM-1 (19). After three days the cells were harvested, washed and analyzed with candidate hybridoma supernatants by indirect immunofluorescence. Briefly, 10⁶ cells were incubated with 50 µl of culture supernatant for 30 min at 4°C. The cells were washed, resuspended in an appropriate dilution of FITC-(Fab₂)' fragments of sheep anti-mouse immunoglobulin antibody (FITC SAMIg) (DDAF, Silenus), and held on ice for 30 min at 4°C. The cells were washed and then fired in 1% formalin in phosphate-buffered saline (PBS). The samples were examined by fluorescence microscopy and 35 putative positives (GMR⁺, ICAM⁻) identified from a total of 390 wells. The cells from these wells were expanded to generate further supernatants for testing and to provide cells for recloning and cryopreservation. Fifteen clones remained positive by immunofluorescence and were further selected by screening in immunodepletion assays (see below).

### (b) Immunodepletion Assays.

COS 7 cells that had been electroporated with the human GM-CSF receptor α-chain cDNA in the CDM8 vector 72 hours previously were lysed, and membranes purified as previously described (20). The membranes were extracted with 1% Triton X-100, centrifuged at 200,000g for 15 min and the supernatant retained as solubilized GM-CSF receptor α-chain.

Solubilized GM-CSF receptor α-chain (50 µl) was preincubated with hybridoma supernatant (100 µl), sheep anti-mouse immunoglobulin (100 µl of a 1/100 dilution) and protein-G Sepharose (100 µl of a 1/5 suspension) in a final volume of 400µl of Hepes-buffered (20 mM, pH 7.4) RPMI medium containing 10% (v/v) foetal calf serum for 60 min at 23°C with rotation. Tubes were then centrifuged at 14,000 g for 10 sec and the supernatants recovered.

Supernatants (50 µl aliquots) were incubated with ¹²⁵I-human GM-CSF (10 µl, -200,000 cpm, specific radioactivity 30,000 cpm/ng) in duplicate, with or without unlabelled human GM-CSF (10 µl of 50 µg/ml) and concanavalin A-Sepharose 4B (30 µl of a 1/4 suspension in 0.1M sodium acetate buffer pH 6.0), for 60 min at 23°C with rotation. The incubation mixture was then layered over 180 µl of foetal calf serum in small centrifuge tubes, centrifuged at 14,000 g for 10 sec and the pellet removed by cutting the tube with a scalpel blade. Specific binding in these pellets was calculated as the binding (cpm) without unlabelled GM-CSF minus the binding in the presence of unlabelled GM-CSF.

The presence of monoclonal antibody against the human GM-CSF receptor α-chain was detected by the ability of the appropriate hybridoma supernatant to deplete soluble human GM-CSF receptor α-chain in the preincubation step (relative to control hybridoma supernatant or antibody), as revealed by a reduction in the specific binding of ¹²⁵I-human GM-CSF detected in the subsequent incubation.

### Example 3 Direct Binding Inhibition Assays.

Solubilized human GM-CSF receptor α-chain (50 µl), monoclonal antibody or hybridoma supernatant (10 µl), ¹²⁵I-human GM-CSF (10 µl, -200,000 cpm), and Concanavalin A-Sepharose 4B (30 µl of a 1/4 suspension) with or without unlabelled human GM-CSF (10 µl of 50 µl/ml), in a total volume of 110 µl of 0.1M NaAcetate buffer pH 6.0 were incubated at 23°C for 60 min with rotation, and specific binding was determined as described above.

### Example 4 Isotype Determination of Monoclonal Antibodies

Supernatants from positive hybridoma clones were isotyped using the Amersham Isotyping Kit (Amersham, Buckinghamshire, England). Monoclonal antibody KI-2B7-17A, along with several other monoclonals, was found to be of the IgG2a type.

### Example 5 Purification of Monoclonal Antibodies

Individual hybridoma cell lines were expanded to 10 litres of culture volume and the supernatants harvested and concentrated 16-fold. The concentrated supernatants were loaded on to a protein-A-Sepharose CL-4B column (5 ml) (Pharmacia, Uppsala, Sweden), and the column was washed extensively with phosphate (20 mM, pH 7.4) buffered 0.15M saline (PBS) until the absorbance at 280 nm was negligible. The column was then eluted with 0.1M glycine HC1 buffer pH 3.0 and fractions collected into 1/10 volume of 1.0M Tris HCl pH 8. The eluates were concentrated and dialyzed extensively against PBS before use.

### Example 6 Radiolabeling of Monoclonal Antibodies

Monoclonal antibodies were radioiodinated with ¹²⁵I either by the modified iodine monochloride method described previously (21), or by the use of ¹²⁵I-Bolton-Hunter reagent (22). Monoclonal antibody KI-2B7-17-A only retained binding activity when iodinated using the Bolton-Hunter reagent, so direct binding studies were performed with this form of the iodinated antibody (specific radioactivity 50,000 cpm/ng).

### Example 7 Immunodepletion Studies

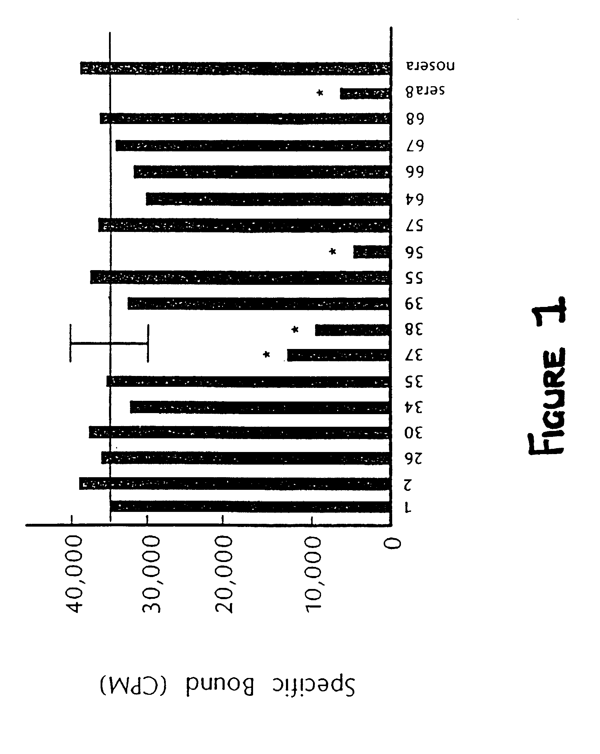

Figure 1 shows the capacity of randomly selected hybridoma supernatants to deplete solubilized human GM-CSF receptor α-chain from solution. After immunodepletion, the solution was tested for residual specific binding of ¹²⁵I-human GM-CSF. Mean specific cpm bound and standard deviation are shown by the horizontal line and error bar in Figure 1 for solutions that had not been immmodepleted. Most hybridoma supernatants did not reduce the specific binding, but supernatants 37, 38 and 56 clearly caused a marked reduction in specific binding, indicating that they contained antibodies that could recognize the human GM-CSF receptor α-chain. In addition the serum from immunized mouse number 8 also contained antibodies which recognized the receptor. This assay allowed the identification of five purified monoclonal antibodies (KI-3G9-25-1, KI-3G3-14-15, KI-2B7-17-A, K3-2E9-4-1 and K3-2C7-16-1), each derived from double cloned hybridoma cell lines that recognized the human GM-CSF receptor α-chain.

### Example 8 Immunological Detection of Human GM-CSF Receptor α-chain on FDA5 Cells

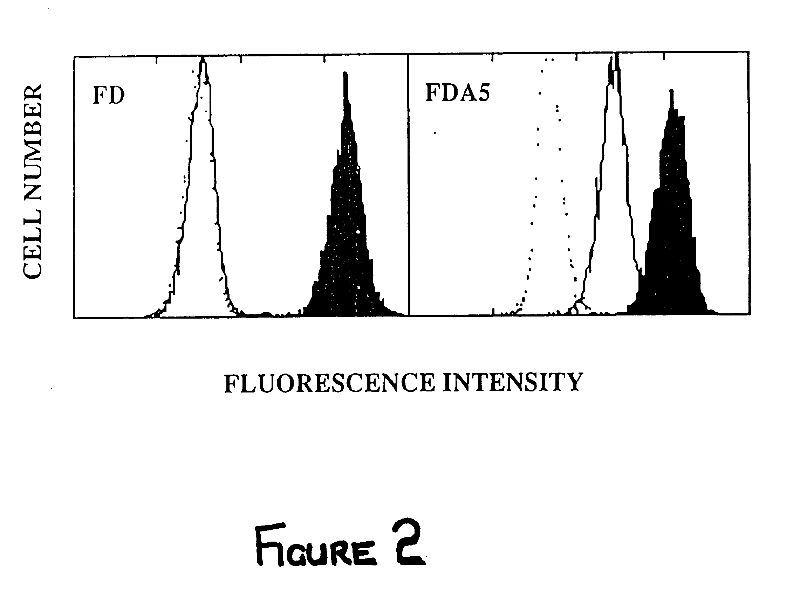

Figure 2 shows that FDA5 cells (17), but not parental FDC-P1 (FD) cells (17), showed significant fluorescence above background when stained with the monoclonal antibodies KI-2B7-17-A and FITC SAMIg and then subjected to flow cytometry (solid line, unfilled). The positive control, indicated in black, shows fluorescence with anti-H2D antibodies. The dotted profile represents fluorescence of cells treated with FITC-SAMIg in the absence of test antibody.

### Example 9 Direct Binding Inhibition Studies

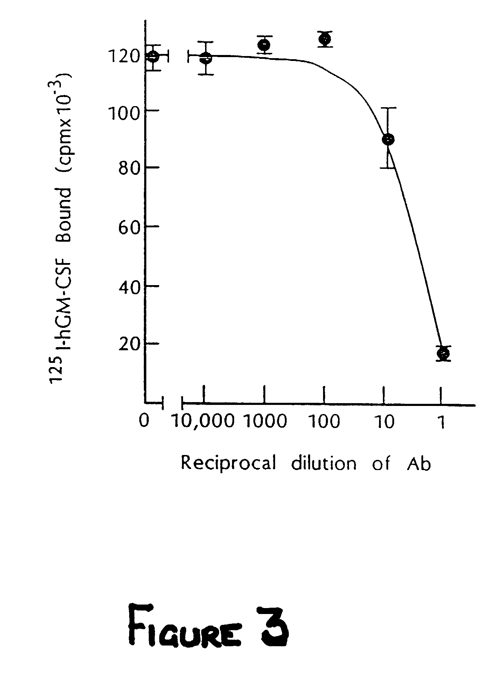

Figure 3 shows that when monoclonal antibody KI-2B7-17-A was co-incubated with solubilized human GM-CSF receptor α-chain and ¹²⁵I-human GM-CSF (with no prior immunodepletion step), the antibody directly interfered with ¹²⁵I-GM-CSF binding to the receptor, as evidenced by the dose-dependent inhibition of the amount of ¹²⁵I-GM-CSF bound to the receptor which was observed.

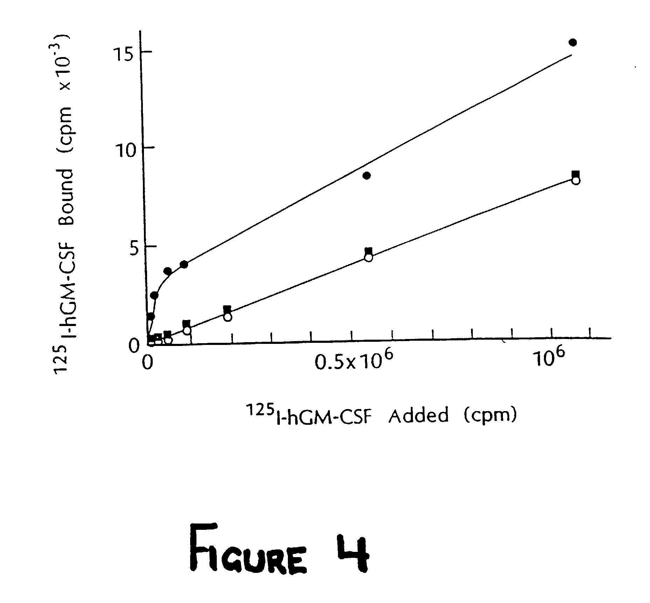

This antibody also completely suppressed specific binding of ¹²⁵I-GM-CSF to GM-CSF receptors on cells of the HL60 promyelocytic leukaemic cell line, which had been induced to differentiate by exposure to 1% dimethylsulphoxide for 5 days, as shown in Figure 4.

When KI-2B7-17-A antibody (10µg/ml) was co-incubated with the cells and ¹²⁵I-human GM-CSF (0-10⁶ cpm, 0-3 ng/ml) it reduced the specific binding (filled squares) to the same extent as unlabelled human GM-CSF (5 µg/ml) (open circle).

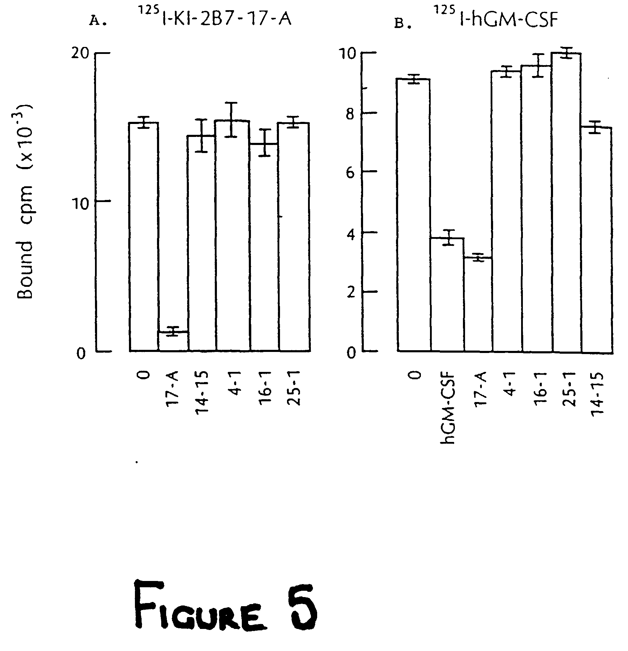

Figure 5 shows that monoclonal antibody KI-2B7-17-A was unique amongst the five purified monoclonal antibodies directed against the human GM-CSF receptor *α*-chain, in that it directly inhibited the binding of ¹²⁵I-human GM-CSF to solubilized receptor. This suggested that KI-2B7-17-A recognized a unique epitope on the GM-CSF receptor α-chain which is involved in GM-CSF binding. This was confirmed by showing that only unlabelled KI-2B7-17-A, and not the other four monoclonal antibodies, could compete for the direct binding of ¹²⁵I-KI-2B7-17-A to human GM-CSF receptor α-chain expressed on COS cells (Figure 5).

### Example 10 Direct Binding of KI-2B7-17-A to Human GM-CSF Receptor

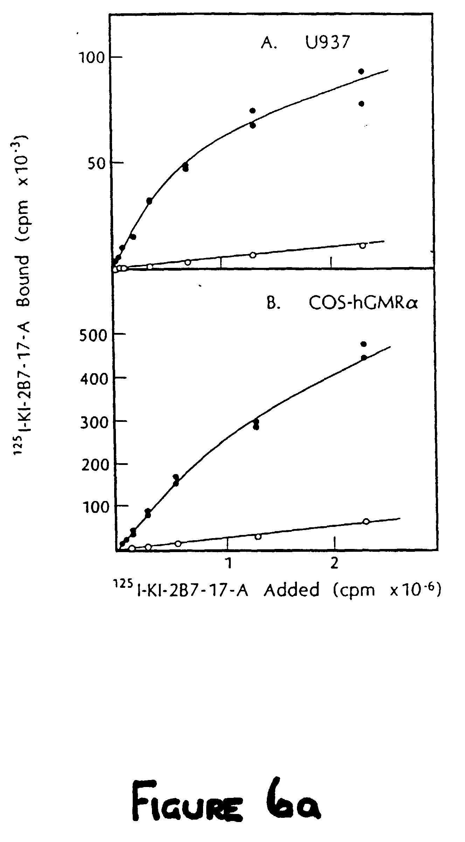

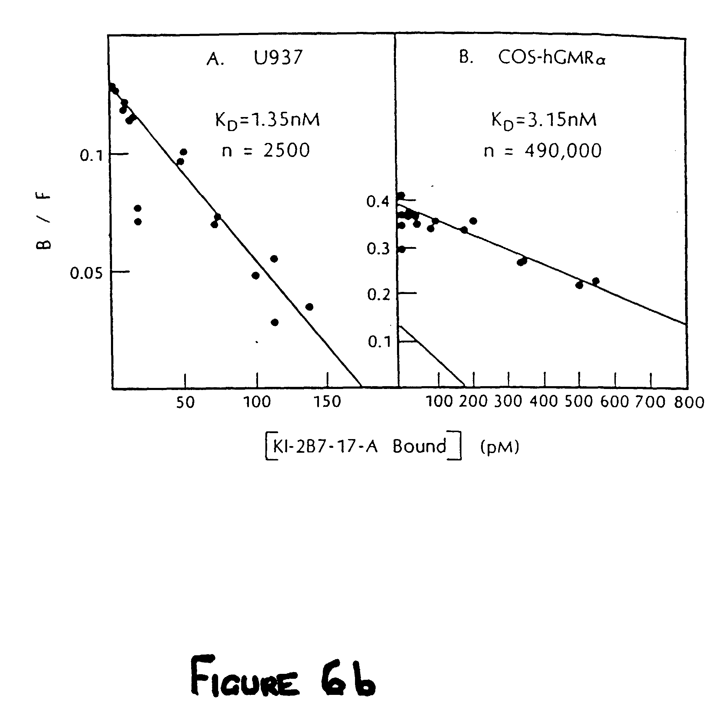

In order to demonstrate the direct binding of the KI-2B7-17-A antibody to human GM-CSF receptor and determine its binding affinity, the antibody was iodinated using ¹²⁵I-Bolton-Hunter reagent. Increasing amounts of ¹²⁵I-KI-2B7-17-A were incubated with U937 human monocytic cells (ATCC CRL 1593) as a source of natural high-affinity human GM-CSF receptor, or with COS cells transfected with human GM-CSF receptor α-chain, and specific binding was determined as the difference in cpm bound in the presence or absence of unlabeled KI-2B7-17-A (12 µg/ml). Figure 6a shows the binding isotherms generated at 4°C, and their transformation into Scatchard plots (20) is shown in Figure 6b. The binding affinity of the antibody to U937 cell GM-CSF receptor was characterized by an equilibrium dissociation constant (K_{D}) of 1.35 nM and 2500 sites/cell. This compares to a K_{D} of 0.41 nM and site number of 1500 sites/cell for ¹²⁵I-human GM-CSF on the same cells (data not shown). A similar affinity of binding of the antibody to transfected COS cells was determined (K_{D}=3.15 nM), although a much higher number of receptors was detected (490,000/cell). The solid line in panel B of Figure 6b is the same as that in panel A, drawn to the same scale for comparative purposes. With these affinity constants KI-2B7-17-A should give 50% inhibition at between 0.1-1 µg/ml, and this accords with the observations in example 11.

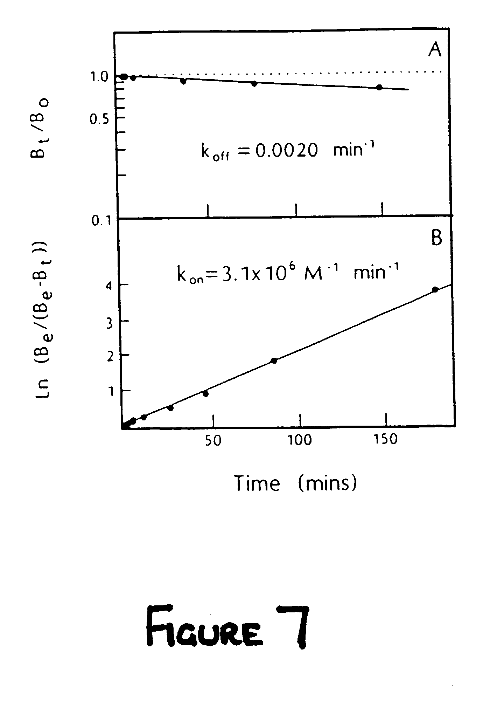

We further characterized the direct binding of ¹²⁵I-KI-2B7-17-A antibody to natural human GM-CSF receptor on U937 cells by measuring its rate of association (kₒₙ) and rate of dissociation (k_{off}) from the receptor (Figure 7). In Part A of this figure, U937 cells (2.2x10⁶ per point) were incubated with ¹²⁵I-KI-2B7-17-A antibody (500,000 cpm/50 µl/point) for at least 1 hr at 4°C. The cells were then rapidly washed and resuspended in binding medium containing 20 µg/m1 of unlabeled antibody. At the indicated time points aliquots of cells were removed, and the specifically bound radioactivity remaining determined as described for other figures. k_{off} was determined from the plot in Figure 7A as 0.002 min⁻¹ (k_{off}=1n 2/t½). In Part B the same number of U937 cells and ¹²⁵I-KI-2B7-17-A antibody were mixed, and aliquots removed at the indicated times at 4°C to determine specifically bound radioactivity (Bₜ). The data were extrapolated to determine the amount bound at infinite time (Be), and the data plotted as 1n[Be/(Be-Bt)] versus time. The slope of the line is kₒₙ[Antibody]+k_{off}, so since the concentration of ¹²⁵I-KI-2B7-17-A was known, kₒₙ could be calculated as 3.1x10⁶ M⁻¹ min⁻¹. The slow dissociation rate of the antibody (0.002 min⁻¹) makes it useful as an inhibitor of the GM-CSF receptor.

### Example 11 Inhibition of the Biological Action of GM-CSF by MonoclonalAntibody KI-2B7-17-A

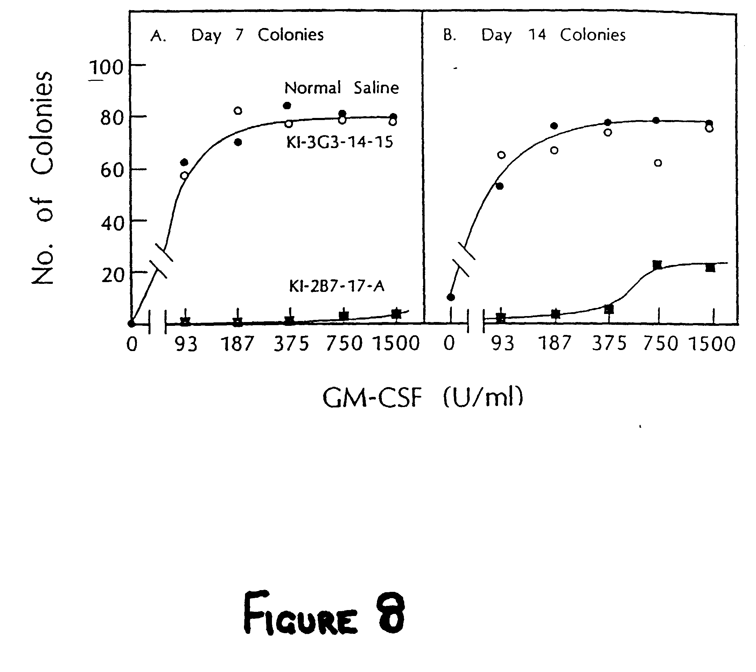

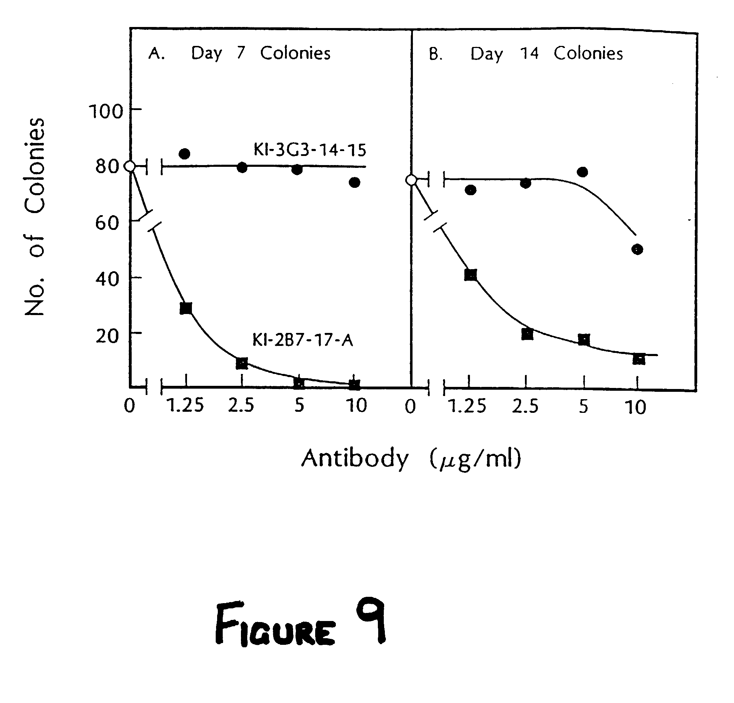

Monoclonal antibody KI-2B7-17-A (filled squares), but not the isotype matched monoclonal antibody KI-3G3-14-15 (open circles), which was obtained from a similar fusion and also recognizes the human GM-CSF receptor α-chain, nor saline (closed circles), was able to inhibit colony formation from human bone marrow cells stimulated by human GM-CSF in semisolid agar cultures *in vitro* (Figure 8). At 10 µg/ml, KI-2B7-17-A almost completely inhibited colony formation induced by up to 1500 U/ml of GM-CSF when cultures were scored at day 7. A similar but less marked inhibition was seen when cultures were scored at day 14. Figure 9 shows that the inhibition of colony formation (stimulated by 400 U/ml of GM-CSF) was dependent on the dose of antibody KI-2B7-17-A, with 50% inhibition occurring at about 1 µg/ml and nearly complete inhibition occurring at about 10 µg/ml.

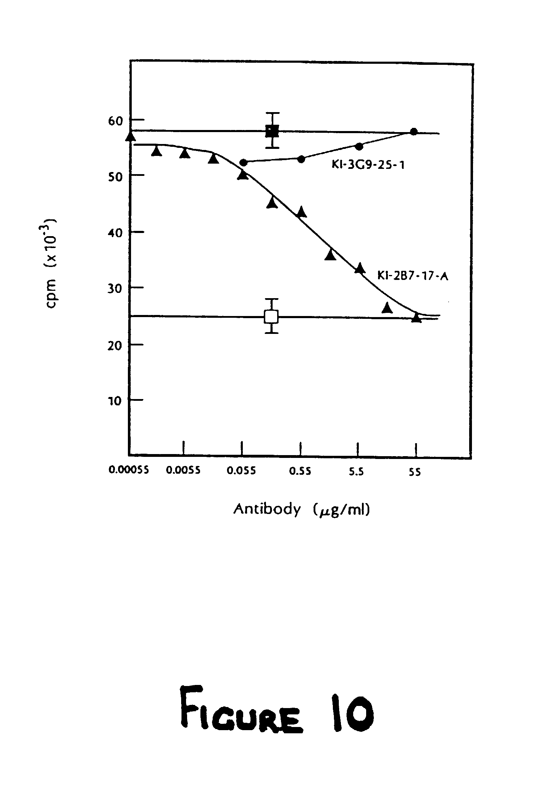

Figure 10 shows that antibody KI-2B7-17-A also inhibited the growth in *vitro* of the human GM-CSF-dependent acute monocytic leukaemia cell line AML-193 (ATCC CRL 9589), as assessed by the inhibition of incorporation of ³H-methyl-thymidine into cellular DNA. 50% inhibition again occurred at 1 µg/ml of antibody, and nearly complete inhibition of GM-CSF-stimulated growth occurred at 10-100 µg/ml. The solid line with a filled square and error bars represents incorporation into cells in the absence of added antibody, while the solid line with open square and error bars represents incorporation in the absence of GM-CSF. No signficant inhibition was observed with the isotype-matched antibody KI-3G9-2Sl (closed circles)

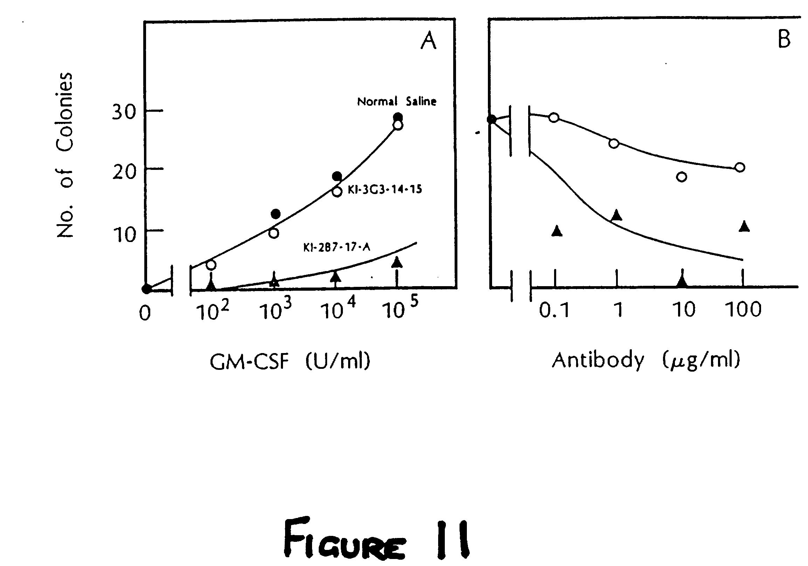

Finally Figure 11 shows that antibody KI-2B7-17-A inhibited the growth *in vitro* in semisolid agar cultures of the FD-A5 cells (mouse FDC-P1 cells transfected with human GM-CSF receptor α-chain) stimulated by human GM-CSF, as assessed by colony formation and colony size. Again 50% inhibition occurred at about 1 µg/ml, and nearly complete inhibition occurred by 10 µg/ml. In the figure only the excess colonies stimulated above the saline control by GM-CSF are shown, with saline addition indicated by closed circles, KI-3G3-14-15 antibody by open circles, and KI-2B7-17-A antibody by closed triangles.

References cited herein are listed on the following pages.

It will be clearly understood that the invention in its general aspects is not limited to the specific details referred to hereinabove.

### REFERENCES

1. Metcalf, D.
   "The molecular control of blood cells"
   Harvard University Press, Cambridge, Mass. 1988
2. Gough, N.M. and Nicola, N.A.
   Granulocyte-macrophage colony-stimulating factor. In "Colony-stimulating factors: molecular and cellular biology." (Dexter, T.M., Garland, J.M. and Testa, N.G., eds).
   Marcel Dekker Inc., H.Y., 1990 111-153
3. Gasson, J.C.
   "Molecular physiology of granulocyte-macrophage colony-stimulating factor"
   Blood, 1991 77 1131-1145.
4. Crosier, P.S., Garnick, M.B. and Clark, S.C.
   Granulocyte-macrophage colony-stimulating factor. In "Human Cytokines: Handbook for Basic and Clinical Research." (Aggarwal, B.B., Gutterman, J.U.,.eds).
   Blackwell, Oxford, 1992 238-252.
5. Gearing, D.P., King, J.A., Gough, N.M. & Nicola, N.A.
   "Expression cloning of a receptor for human granulocyte-macrophage colony-stimulating factor"
   EMBO J., 1989 8 3667-3676.
6. Kitamura, T., Sato, N., Arai, K. and Miyajima, A.
   "Expression cloning of the human IL-3 receptor cDNA reveals a shared beta subunit for the human IL-3 and GM-CSF receptors"
   Cell, 1991 66 1165-1184.
7. Tavernier, J., Devos, R., Cornelis, S., Tuypens, T., Van der Heyden, J., Fiers, W. and Plaetinck, G.
   "A human high affinity interleukin-5 receptor (IL5R) is composed of an IL5-specific alpha chain and a beta chain shared with the receptor for GM-CSF"
   Cell, 1991 66 1175-1184.
8. Kitamura, T., Hayashida, K., Sakamaki, K., Yokota, T., Arai, K. and Mijajima, A.
   "Reconstitution of functional receptor for human granulocyte-macrophage colony-stimulating factor (GM-CSF): evidence that the protein encoded by the AIC2B cDNA is a subunit of the murine GM-CSF receptor"
   Proc. Natl. Acad. Sci. (USA), 1991 88 5082-5086.
9. Lang, R.A., Metcalf, D., Cuthbertson, R.A., Lyons, I., Stanley, E., Kelso, A., Kannourakis, G., Williamson, D.J., Klintworth, G.K., Gonda, T.J. and Dunn, A.R.
   "Transgenic mice expressing a hemopoietic growth factor gene (GM-CSF) develop accumulations of macrophages, blindness, and a fatal syndrome of tissue damage"
   Cell, 1987 51 675-686.
10. Johnson, G.R., Gonda, T.J., Metcalf, D., Hariharan, I.K. and Cory, S.
   "A lethal myeloproliferative syndrome in mice transplanted with bone marrow cells infected with a retrovirus expressing granulocyte-macrophage colony stimulating factor"
   EMBO J., 1989 8 441-448.
11. Grant, S.M. and Heel, R.C.
   "Recombinant granulocyte-macrophage colony-stimulating factor (rGM-CSF): A review of its pharmacological properties and prospective role in the management of myelosuppression.
   Drugs, 1992 43 516-560.
12. Begley, C.G., Metcalf, D. and Nicola, N.A.
   "Primary human myeloid leukemia cells: comparative responsiveness to proliferative stimulation by GM-CSF or G-CSF and membrane expression of CSF receptors"
   Leukemia, 1989 1 1-4.
13. Griffin, J.D., Young, D., Herrmann, F., Wiper, D., Wagner, K. and Sabbath, K.D.
   "Effects of recombinant human GM-CSF on proliferation of clonogenic cells in acute myeloblastic leukemia"
   Blood, 1986 67 1448-1453.
14. Kelleher, C.A., Wong, G.G., Clark, S.C., Schendel, P.F., Minden, M.D. and McCulloch, E.A.
   "Binding of iodinated recombinant human GM-CSF to the blast cells of acute myeloblastic leukemia"
   Leukemia, 1988 2 211-215.
15. Onetto-Pothier, N., Aumont, K., Haman, A., Bigras, C., Wong, G.G., Clark, S.C., De Lean, A. and Hoang, T. "Characterization of GM-CSF receptor on the blast cells of acute myeloblastic leukemia"
   Blood, 1990 75 59-66.
16. Baldwin, G.C., Gasson, J.C., Kaufman, S.E., Quan, S.G., Williams, R.E., Avalos, B.R., Gazdar, A.F., Golde, D.W. and Di Persio, J.F.
   "Nonhematopoietic tumor cells express functional GM-CSF receptors"
   Blood, 1989 73 1033-1037.
17. Metcalf, D., Nicola, N.A., Gearing, D.P. and Gough, N.M.
   "Low-affinity placenta-derived receptorsfor human granulocyte-macrophage colony-stimulating factor can deliver a proliferative signal to murine hemopoietic cells"
   Proc. Natl. Acad. Sci. (USA), 1990 8 4670-4674.
18. KÖhler, G. and Milstein, C.
   "Derivation of specific antibody-producing tissue culture and tumor lines by cell fusion"
   Eur. J. Immmol., 1976 6 511-519.
19. Simmons, D., Makgoba, M.W. and Seed, B.
   "ICAM, an adhesion ligand of LFA-1, is homologous to the neural cell adhesion molecule NCAM"
   Nature, 1976 331 624-627.
20. Nicola, N.A. and Cary, D.
   "Affinity conversion of receptors for colony-stimulating factors: properties of solubilized receptors"
   Growth Factors, 1992 6 119-129.
21. Nicola, N.A. and Metcalf, D.
   "Binding, internalization and degradation of ¹²⁵I-multipotential colony-stimulating factor (interleukin-3) by FDCP-1 cells"
   Growth Factors, 1988 1 29-39.
22. Bolton, A.E. and Hunter, W.M.
   "The labeling of proteins to high specific radioactivities by conjugation to a ¹²⁵I-containing acylating agent: application to the radioimmunoassay.
   Biochem. J., 1973 133 529-539.

## Claims

1. A monoclonal antibody, fragment or derivative thereof, specific for human GM-CSF receptor (hGM-CSF receptor), said antibody, fragment or derivative having the ability to inhibit the biological activity of hGM-CSF, and said antibody being able to bind to both the high-affinity and to the low-affinity hGM-CSF receptor.

2. A monoclonal antibody, fragment or derivative as claimed in claim 1, wherein the biological activity is GM-CSF dependent growth.

3. A monoclonal antibody, fragment or derivative as claimed in claim 1 or 2 having the ability to inhibit the binding of GM-CSF to the high-affinity hGM-CSF receptor.

4. Monoclonal antibody KI-2B7-17-A from hybridoma cell line KI-2B7-17-A, as deposited in the European Collection of Animal Cell Cultures on 23 September 1992 under Accession Number 92092317 or fragment or derivative thereof having the ability to inhibit the biological activity of hGM-CSF.

5. A monoclonal antibody, fragment or derivative as claimed in any one of claims 1 to 4 produced by genetic engineering methods.

6. A therapeutic composition comprising a monoclonal antibody, fragment or derivative as defined in any one of claims 1 to 5 together with at least one pharmaceutically-acceptable carrier.

7. A hybridoma cell line producing an antibody as defined in any one of claims 1 to 5.

8. Hybridoma cell line KI-2B7-17-A, as deposited in the European Collection of Animal Cell Cultures on 23 September 1992 under Accession Number 92092317.

9. The use of an antibody, fragment or derivative as defined in any one of claims 1 to 5 for the preparation of a medicament for treating a GM-CSF dependent condition.

10. The use as claimed in claim 9 wherein the GM-CSF dependent condition is acute or chronic myeloid leukaemia or another leukaemia which is influenced by GM-CSF.

11. The use of an antibody, fragment or derivative as defined in any one of claims 1 to 5 for the preparation of a medicament for treating the side-effects of treatment with GM-CSF.

12. A method of screening of selective antagonists to GM-CSF, comprising the step of utilising an antibody, fragment or derivative as defined in any one of claims 1 to 5, to identify the specific epitope on the GM-CSF receptor recognised by said antibody, fragment or derivative and testing putative antagonists of GM-CSF action for the ability to bind to said epitope and inhibit the biological activity of GM-CSF.

## Patentansprüche

1. Monoklonaler Antikörper, Fragment oder Derivat desselben, spezifisch für den menschlichen GM-CSF-Rezeptor (hGM-CSF-Rezeptor), wobei der Antikörper, das Fragment oder das Derivat die Fähigkeit besitzt, die biologische Aktivität des hGM-CSF zu hemmen und wobei der Antikörper sowohl an den hochaffinen als auch an den schwachaffinen hGM-CSF-Rezeptor binden kann.

2. Monoklonaler Antikörper, Fragment oder Derivat nach Anspruch 1, wobei die biologische Aktivität das von GM-CSF abhängige Wachstum ist.

3. Monoklonaler Antikörper, Fragment oder Derivat nach Anspruch 1 oder 2, mit der Fähigkeit, die Bindung des GM-CSF an den hochaffinen hGM-CSF-Rezeptor zu hemmen.

4. Monoklonaler Antikörper K1-2B7-17-A aus der Hybridomzellinie KI-2B7-17-A, wie am 23. September 1992 unter der Zugriffsnummer 92092317 bei der Europäischen Sammlung für Tierzellkulturen (European Collection of Animal Cell Cultures) hinterlegt, oder Fragment oder Derivat desselben, mit der Fähigkeit, die biologische Aktivität des hGM-CSF zu hemmen.

5. Monoklonaler Antikörper, Fragment oder Derivat nach einem der Ansprüche 1 bis 4, der oder das durch gentechnologische Verfahren hergestellt wurde.

6. Therapeutische Zusammensetzung, die einen monoklonalen Antikörper, ein Fragment oder ein Derivat, wie in einem der Ansprüche 1 bis 5 definiert, zusammen mit mindestens einem pharmazeutisch verträglichen Träger umfaßt.

7. Hybridornzellinie, die einen Antikörper, wie in einem der Ansprüche 1 bis 5 definiert, produziert.

8. Hybridomzellinie KI-2B7-17-A, wie am 23. September 1992 unter der Zugriffsnummer 92092317 bei der Europäischen Sammlung für Tierzellkulturen hinterlegt.

9. Verwendung eines Antikörpers, Fragments oder Derivats, wie in einem der Ansprüche 1 bis 5 definiert, zur Herstellung eines Arzneimittels zur Behandlung eines vom GM-CSF abhängigen Zustands.

10. Verwendung nach Anspruch 9, wobei der von GM-CSF abhängige Zustand akute oder chronische myeloische Leukämie oder eine andere durch GM-CSF beeinflußte Leukämie ist.

11. Verwendung eines Antikörpers, Fragments oder Derivats, wie in einem der Ansprüche 1 bis 5 definiert, zur Herstellung eines Arzneimittels zur Behandlung der Nebenwirkungen einer Behandlung mit GM-CSF.

12. Screening-Verfahren für selektive Antagonisten des GM-CSF, das folgenden Schritt umfaßt: Verwenden eines Antikörpers, Fragments oder Derivats, wie in einem der Ansprüche 1 bis 5 definiert, zur Identifizierung des spezifischen Epitops des GM-CSF-Rezeptors, das von dem Antikörper, Fragment oder Derivat erkannt wird, und Untersuchen mutmaßlicher Antagonisten der GM-CSF-Wirkung auf die Fähigkeit, an das Epitop zu binden und die biologische Aktivität des GM-CSF zu hemmen.

## Revendications

1. Anticorps monoclonal, fragment ou dérivé de celui-ci spécifique pour un récepteur pour GM-CSF humain (récepteur pour hGM-CSF), le dit anticorps, fragment ou dérivé ayant l'aptitude à inhiber l'activité biologique de hGM-CSF, et le dit anticorps étant capable de se fixer à la fois au récepteur pour hGM-CSF de haute affinité et au récepteur hGM-CSF de faible affinité.

2. Anticorps monoclonal, fragment ou dérivé suivant la revendication 1, dans lequel l'activité biologique est une croissance GM-CSF-dépendante.

3. Anticorps monoclonal, fragment ou dérivé suivant les revendications 1 ou 2, ayant l'aptitude à inhiber la liaison de GM-CSF au récepteur pour hGM-CSF de haute affinité.

4. Anticorps monoclonal KI-2B7-17-A provenant de la lignée cellulaire d'hybridome KI-2B7-17-A, telle qu'elle a été déposée à l'European Collection of Animal Cell Cultures, le 23 septembre 1992 sous le numéro d'accès 92092317 ou fragment ou dérivé de celui-ci ayant l'aptitude à inhiber l'activité biologique de hGM-CSF.

5. Anticorps monoclonal, fragment ou dérivé suivant l'une quelconque des revendications 1 à 4, produit par des procédures de génie génétique.

6. Composition thérapeutique comprenant un anticorps monoclonal, un fragment ou un dérivé tel que défini dans l'une quelconque des revendications 1 à 5 avec au moins un support acceptable du point de vue pharmaceutique.

7. Lignée cellulaire d'hybridome produisant un anticorps tel que défini dans l'une quelconque des revendications 1 à 5.

8. Lignée cellulaire d'hybridome KI-2B7-17-A, telle qu'elle a été déposée à l'European Collection of Animal Cell Cultures, le 23 septembre 1992 sous le numéro d'accès 92092317.

9. Utilisation d'un anticorps, d'un fragment ou d'un dérivé tel que défini dans l'une quelconque des revendications 1 à 5 pour la préparation d'un médicament pour le traitement d'un état GM-CSF-dépendant.

10. Utilisation suivant la revendication 9, dans laquelle l'état GM-CSF-dépendant est une leucémie myéloïde aiguë ou chronique ou une autre leucémie qui est influencée par GM-CSF.

11. Utilisation d'un anticorps, d'un fragment ou d'un dérivé tel que défini dans l'une quelconque des revendications 1 à 5 pour la préparation d'un médicament pour le traitement des effets secondaires d'un traitement avec GM-CSF.

12. Procédé de criblage d'antagonistes sélectifs de GM-CSF, comprenant l'étape d'utilisation d'un anticorps, d'un fragment ou d'un dérivé tel que défini dans l'une quelconque des revendications 1 à 5 pour identifier l'épitope spécifique sur le récepteur pour GM-CSF reconnu par le dit anticorps, fragment ou dérivé et de test d'antagonistes putatifs de l'action de GM-CSF pour l'aptitude à se lier au dit épitope et à inhiber l'activité biologique de GM-CSF.
